# EUROPEAN PATENT SPECIFICATION

(11) **EP 3 712 617 B1**
(45) Date of publication and mention of the grant of the patent: **26.01.2022**
(21) Application number: 19163598.6
(22) Date of filing: 19.03.2019
(51) Int. Cl.: G01N 33/53, A61K 39/00, A61K 35/17, G01N 33/537, G01N 33/542, G01N 33/569

(54) **METHOD FOR PROVIDING PERSONALIZED CELLS WITH CHIMERIC ANTIGEN RECEPTORS (CAR)**
VERFAHREN ZUR BEREITSTELLUNG PERSONALISIERTER ZELLEN MIT CHIMÄREN ANTIGENREZEPTOREN (CAR)
PROCÉDÉ DE FOURNITURE DE CELLULES PERSONNALISÉES COMPORTANT DES RÉCEPTEURS D'ANTIGÈNES CHIMÉRIQUES (CAR)

(43) Date of publication of application: 23.09.2020
(73) Proprietor: Miltenyi Biotec B.V. & Co. KG, 51429 Bergisch Gladbach (DE)
(72) Inventor: BOSIO, Andreas, 50933 Köln (DE); HARDT, Olaf, 51061 Köln (DE); ECKARDT, Dominik, 51429 Bergisch Gladbach (DE); HERBEL, Christoph, 51491 Overath (DE)
(74) Representative: Kisters, Michael Marcus

(56) References cited:
- EP-A1- 3 311 832
- US-A1- 2017 107 285
- CHEN CHENG ET AL: "Development of T cells carrying two complementary chimeric antigen receptors against glypican-3 and asialoglycoprotein receptor 1 for the treatment of hepatocellular carcinoma", CANCER IMMUNOLOGY, IMMUNOTHERAPY, SPRINGER, BERLIN/HEIDELBERG, vol. 66, no. 4, 29 December 2016 (2016-12-29), pages 475-489, XP036192310, ISSN: 0340-7004, DOI: 10.1007/S00262-016-1949-8 [retrieved on 2016-12-29]
- XIAOLU HAN ET AL: "Masked Chimeric Antigen Receptor for Tumor-Specific Activation", MOLECULAR THERAPY, vol. 25, no. 1, 1 January 2017 (2017-01-01), pages 274-284, XP055613751, ISSN: 1525-0016, DOI: 10.1016/j.ymthe.2016.10.011

## Description

### Field of invention

The present invention relates to a method for selecting antigens exposed on cancer cells but not on healthy cells of an individual in order to provide cells with appropriate chimeric antigen receptors (CAR).

### Background

Cancer is a broad group of diseases involving unregulated cell growth. In cancer, cells divide and grow uncontrollably, forming malignant tumors, and invading nearby parts of the body. The cancer may also spread to more distant parts of the body through the lymphatic system or bloodstream.

The technology of chimeric antigen receptor (CAR) may provide a promising approach for adoptive cell immunotherapy for cancer. Commonly, CARs comprise a single chain fragment variable (scFv) of an antibody specific for a tumor associated antigen (TAA) coupled via hinge and transmembrane regions to cytoplasmic domains of T-cell signaling molecules.

By introducing a chimeric antigen receptor (CAR) into T Cells (hereinafter referred to as CAR T Cells) T cells would be generated which are tailored to recognize tumor cells as "non-self cells" and therefore help the immune system to eradicate tumor cells. For example, CAR T Cells may contact a surface molecule of the target cell which then triggers the activation of the CAR T cell and leads to the lysis of the target cell.

Manufacturing and use of CAR T cells is known and disclosed for example in US2017107285 and EP3311832.

CAR T cells provided with two antigen receptors are known from CHEN CHENG ET AL, "Development of T cells carrying two complementary chimeric antigen receptors against glypican-3 and asialoglycoprotein receptor 1 for the treatment of hepatocellular carcinoma", CANCER IMMUNOLOGY, IMMUNOTHERAPY, SPRINGER, BERLIN/HEIDELBERG, vol. 66, no. 4, doi:10.1007/S00262-016-1949-8, ISSN 0340-7004, (20161229), pages 475 - 489

Further, XIAOLU HAN ET AL, disclose in "Masked Chimeric Antigen Receptor for Tumor-Specific Activation", MOLECULAR THERAPY, (20170101), vol. 25, no. 1, ISSN 1525-0016, pages 274 - 284 the use of CAR cells which are blocked on demand to prevent the CAR cell from engaging non-tumor cells.

However, developing effective CAR T Cells face amongst others the following issues:
i) One (1) surface molecule is sometimes not specific enough, it can be expressed on non-tumor cells as well (safety problem)
ii) a surface molecule is not expressed on all tumor cells, surviving (escaping) tumor cells lead to relapse
iii) tumor cells shut down expression of surface molecule and escape therapy
iv) expression level of antigen or affinity of single binder might be too low to allow for efficient T cell activation
iv) strong heterogeneity of antigen expression within tumor samples as well in between tumor samples require personalized profiling of tumor marker expression (kinetics), some markers even appear either on all or none of the tumor cells of tumor from the same type

A potential way to address the above mentioned problems is to use more than one surface molecule as target, i.e. so called "DUAL CAR T Cells" detecting more than one target on a given cell.

The "DUAL CAR T Cells" approach has not been established appropriately so far, especially given the fact that the more complex a combination of multiple targets needs to be to appropriately hit a tumor the less likely it is that this certain combination will suit more than one or a few patients (need for personalization of the therapy). Moreover if the tumor further grows after a first therapy, it is very likely that a new combination of CARs has to be found to further eradicate the tumor.

Further, in order to perform a personalized phenotyping of the tumor marker expression for an individual patient, combinations of CARs and CAR T cells need to be developed in an acceptable time frame which is dictated by the patient and the indication. In clinical praxis, an optimal time frame would be a couple of weeks.

Accordingly, the success of any immunotherapy for cancer based on CAR cells will rely on a fast and reliable selection of antigens specific for the respective tumor cells.

It has been found that a distinct group of cell surface antigens is expressed on human cancer cells, but not or to a lower level on non-malignant cells. These antigens (also referred to as "markers") can be used to identify and/or mark and/or destroy and/or disable escape mechanisms of such cancer cells via ligands that specifically bind to the markers.

Object of the invention was therefore to provide a method to select antigens specific for cancer cells, which are not expressed on healthy tissue in order to engineer cells which then kill/lyse cancer cells without attacking non-tumor cells.

### Summary of the invention

A first object of the invention is a process for providing a cell comprising a chimeric antigen receptor (CAR) specific for one or more target antigens exposed on tumor cells characterized by providing a cell sample comprising tumor and non-tumor cells and repeating the steps
- contacting the cell tissue with a conjugate comprising a fluorescent moiety and an antigen recognizing moiety (hereinafter referred to as "conjugate")
- removing unbound conjugate from the cell tissue and detecting cells bound to the conjugate by the fluorescence radiation emitted by the fluorescent moieties of the first conjugates
- erasing the fluorescence radiation emitted by the fluorescent moieties of the conjugates

with conjugates comprising a fluorescent moiety and antigen recognizing moieties recognizing different antigens
until identifying at least two conjugates provided with antigen recognizing moieties recognizing different antigens, binding to tumor cells but essentially not to non-tumor cells and
providing cells or a cell sample or a cell population with the identified at least two antigen recognizing moieties, preferable 2,3,4 or 5, as chimeric antigen receptor (CAR).

The cell comprising a chimeric antigen receptor (CAR) may be selected from the group consisting of T-cells, NK-cells, or cells engineered to destroy (lyse) other cells when triggered by binding to the other cell.

Since a tumor might be destroyed even if only a part of the tumor cells are identified or attacked, it is not necessary that the conjugates identified bind to all tumor cells. Preferable, the process of the invention is performed until identifying at least two conjugates provided with antigen recognizing moieties recognizing different antigens, binding to at least 1%, more preferred at least 10% and most preferred at least 30 % of the tumor cells.

While it is desired that the conjugates bind to 0% of the non-tumor cells, it is acceptable for therapy that a certain amount of non-tumor cells are targeted. Preferable, the process of the invention is performed until identifying at least two conjugates, at least provided with antigen recognizing moieties recognizing different antigens, binding to tumor cells and to at most 10 %, more preferred to at most 5% and most preferred to at most 1% of the non-tumor cells of the same type of tissue or organ. The term "same type of tissue or organ" refers for example to pancreas or liver.

The process of the invention enables the rapid identification of one or more sets of two conjugates provided with antigen recognizing moieties recognizing different antigens and subsequent one or more different cells with the identified at least two with antigen recognizing moieties as chimeric antigen receptor (CAR). Providing more than one set of conjugates and more than one CAR cell enhances that chances of destroying tumor cells and/or targeting tumor cells downregulating antigens to escape being targeted.

### Brief description of the drawings

- Fig. 1: shows the general domains of a chimeric antigen receptor (CAR)
- Fig. 2: shows general building concepts of chimeric antigen receptors
- Fig. 3: shows schematic the function of a CAR-T cell provided with an AND logic
- Fig. 4: shows schematic the function of a CAR-T cell provided with an NOT logic
- Fig 5: shows the staining of one and the same pancreas cancer specimen with a plurality of conjugates comprising a fluorescent moiety and an antigen recognizing moiety to detect marker which are expressed on pancreatic cancer cells.
- Fig 6: shows a comparative labelling of selected markers on pancreatic cancer cells and healthy tissue to detect combinations of markers which are specifically labelling pancreatic cancers cells but not healthy tissue.
- Fig 7: shows the labelling of high-grade serous ovarian carcinoma to identify potential target candidates.
- Fig 8: shows a comparative expression analysis of selected markers on high-grade serous ovarian carcinoma and healthy tissues to detect combinations of markers which are specifically labelling ovarian carcinoma cells but not healthy tissue.

### Definitions

The term "Chimeric antigen receptor" or "CAR" refers to engineered cell with are provided with an new specificity originating from different sources.

The term "tumor" is known in medicine as "neoplasm". Not all tumors are cancerous; benign tumors do not invade neighboring tissues and do not spread throughout the body. The process of the invention may be applied on both, neoplasms and benign tumors.

The term "cancer" refers to a broad group of diseases involving unregulated cell growth. In cancer, cells (cancerous cells) divide and grow uncontrolled, forming malignant tumors, and invading nearby parts of the body. The cancer may also spread to more distant parts of the body through the lymphatic system or bloodstream.

The terms "binder" or "specifically binds" or "specific for" with respect to an antigen-binding domain of a ligand like an antibody, of a fragment thereof or of a CAR refer to an antigen-binding domain which recognizes and binds to a specific antigen, but does not substantially recognize or bind other molecules in a sample. An antigen-binding domain that binds specifically to an antigen from one species may bind also to that antigen from another species. This cross-species reactivity is not contrary to the definition of that antigen-binding domain as specific. An antigen-binding domain that specifically binds to an antigen may bind also to different allelic forms of the antigen (allelic variants, splice variants, isoforms etc.). This cross reactivity is not contrary to the definition of that antigen-binding domain as specific.

The terms "engineered cell" and "genetically modified cell" as used herein can be used interchangeably. The terms mean containing and/or expressing a foreign gene or nucleic acid sequence which in turn modifies the genotype or phenotype of the cell or its progeny. Especially, the terms refers to cells, preferentially T cells which are manipulated by recombinant methods well known in the art to express stably or transiently peptides or proteins which are not expressed in these cells in the natural state. For example, T cells are engineered to express an artificial construct such as a chimeric antigen receptor on their cell surface. For example, the sequences encoding the CAR may be delivered into cells using a retroviral or lentiviral vector.

The term "target" as used herein refers to an antigen or epitope associated with a cell that should be recognized specifically by an antigen binding domain, e.g. an antigen binding domain of an antibody or of a CAR. The antigen or epitope for antibody recognition can be bound to the cell surface but also be secreted, part of the extracellular membrane, or shed from the cell.

The term "antibody" as used herein refers to polyclonal or monoclonal antibodies and fragments thereof, which can be generated by methods well known to the person skilled in the art. The antibody may be of any species, e.g. mice, rat, sheep, human. For therapeutic purposes, if non-human antigen binding fragments are to be used, these can be humanized by any method known in the art. The antibodies may also be modified antibodies (e.g. oligomers, reduced, oxidized and labeled antibodies).

The term "killer cell" as used herein refers to a cell that can kill/destroy/lyse another cell, e.g. a cancer cell. Most frequently, T cells, NK cells, dendritic cells and macrophages can be used as killer cells.

The term "engineered killer cell" as used herein refers to a killer cell that is genetically modified to allow for the specific killing of a target cell, e.g. a cell modified with a CAR against a target to kill tumor cell expressing the respective target.

### Detailed description of the invention

The process of the invention enables rapid identification of suitable sets of conjugates and subsequent rapid engineering of appropriate CAR cells to destroy the tumor cells of a cell sample. The cell sample comprising tumor and non-tumor cells preferably origins from the same tissue or organ from the same patient.

In order to distinguish tumor and non-tumor cells in a cell sample, a skilled person like a pathologist may decide according to criteria known or *lege artis* in oncology whether the cell sample contains a tumor and if yes, what parts of the cell sample. Such criteria are for example the guidelines published by the Arbeitsgemeinschaft der Wissenschaftlichen Medizinischen Fachgesellschaften (AWMF) and may involve e.g. the morphology of cells, structure of tissue, ploidity, biomarker expression (such as Her2, p53, BRAC1, APC, EGFR etc).

The cell sample is then contacted with a plurality of binders to characterize the recognition pattern and to select binders specific for the tumor cells and binders specific for non-tumor cells. The thus identified binders can be used for subsequent therapy as specified later.

In a variant of the process, at least two conjugates provided with antigen recognizing moieties recognizing different antigens bind to at least 20% of the tumor cells and less than 5% of the non-tumor cells of the cell sample. Preferable, at least two conjugates provided with antigen recognizing moieties recognizing different antigens bind to at least 50% of the tumor cells and less than 5% of the non-tumor cells of the cell sample. More preferable, at least two conjugates provided with antigen recognizing moieties recognizing different antigens bind to at least 70% of the tumor cells and less than 5% of the non-tumor cells of the cell sample.

In another variant, characterized in that the at least two conjugates provided with antigen recognizing moieties destroying at least 10 fold, preferable at least 50 fold, more preferable at least 100 fold more tumor cells than non-tumor cells of the cell sample.

The process of invention involved identifying at least two conjugates provided with antigen recognizing moieties recognizing different antigens, binding to tumor cells but essentially not to non-tumor cells.

If the CAR is provided with at least two antigen recognizing moieties, the CAR may be triggered to perform in different logic gates. The logic gates have been described in the literature as "AND"-, "NOT"-, "OR"- type CAR cells. As disclosed in more detail later, in the process of the invention, the at least two antigen recognizing moieties are provided as AND-, OR- or NOT-type and/or the at least two with antigen recognizing moieties are provided as ADAPTER-type. The at least two antigen recognizing moieties in all these variants are provided as chimeric antigen receptor (CAR).

### Chimeric Antigen Receptor (CAR)

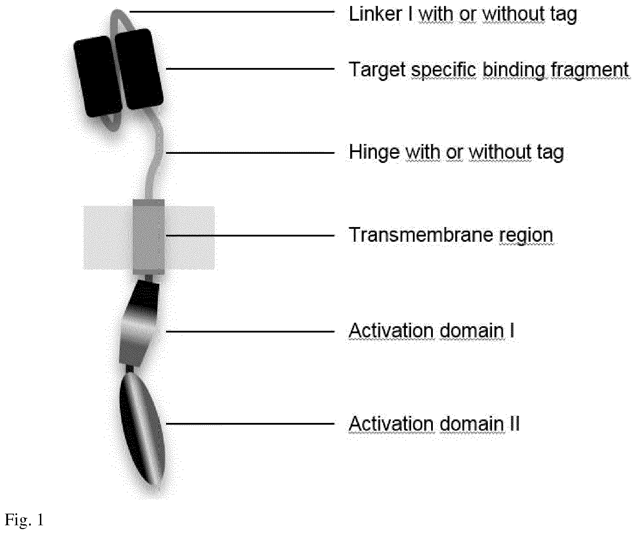

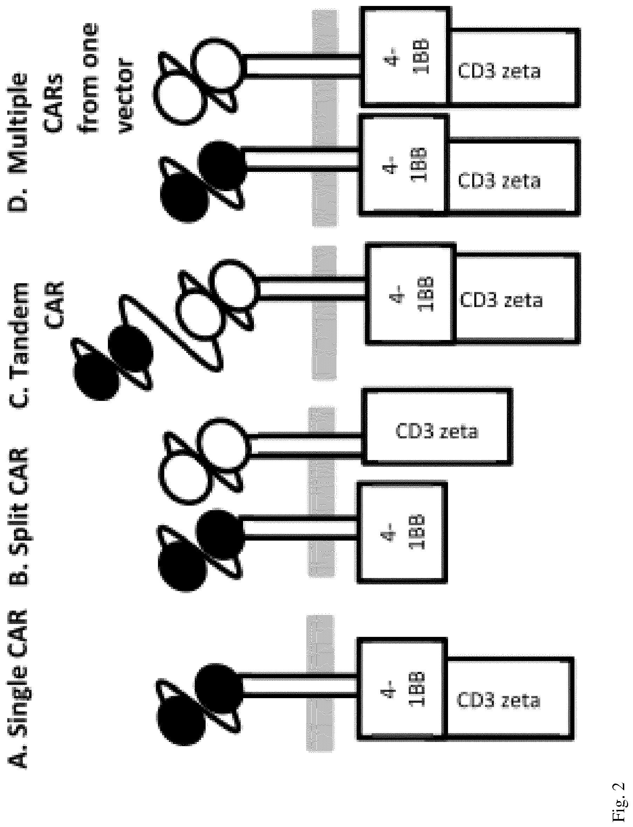

As shown in general in Fig. 1 and 2, a chimeric antigen receptor (CAR) may comprise an extracellular domain comprising the antigen binding domain, a transmembrane domain and an intracellular signaling domain. The extracellular domain may be linked to the transmembrane domain by a linker. The extracellular domain may also be linked to a signal peptide.

The term "signal peptide" refers to a peptide sequence that directs the transport and localization of the protein within a cell, e.g. to a certain cell organelle (such as the endoplasmic reticulum) and/or the cell surface.

The term "antigen binding domain" refers to the region of the CAR that specifically binds to an antigen (and thereby is able to target a cell containing an antigen). The CARs obtained by the invention may comprise one or more antigen binding domains. Generally, the antigen binding domain on the CAR are extracellular. The antigen binding domain may comprise an antibody or a fragment thereof. The antigen binding domain may comprise, for example, full length heavy chain, Fab fragments, single chain Fv (scFv) fragments, divalent single chain antibodies or diabodies. Any molecule that binds specifically to a given antigen such as affibodies or ligand binding domains from naturally occurring receptors can be used as an antigen binding domain. Often the antigen binding domain is a scFv. Normally, in a scFv the variable portions of an immunoglobulin heavy chain and light chain are fused by a flexible linker to form a scFv. Such a linker may be for example the "(G₄/S₁)₃-linker".

The transmembrane domain of the CAR may for example comprise a sequence of the transmembrane domains of 4-1BB, CD8 and/or CD28. Further, an activating intracellular signaling domain may be present comprising a sequence of the intracellular signaling domains of CD28, CD137 or CD3zeta.

In alternative to an activating intracellular signaling domain, the CAR may comprise an inhibiting intracellular signaling domain having a sequence of the intracellular signaling domains such as PD1 or CTLA4.

In a preferred variant of this embodiment, the chimeric antigen receptor (CAR) comprises an antigen binding domain specific for CD20 without an additional antigen binding domain or additional CAR, wherein the antigen binding domain is conjugated to one transmembrane domains and one or more signaling domains. This variant is shown by way of example in Fig. 2 A.

In some instances, it is beneficial for the antigen binding domain to be derived from the same species in which the CAR will be used in. For example, if the CAR is intended to be used therapeutically in humans, it may be beneficial for the antigen binding domain of the CAR to comprise a human or humanized antibody or fragment thereof. Human or humanized antibodies or fragments thereof can be made by a variety of methods well known in the art.

The terms "spacer" or "hinge" as used herein refers to the hydrophilic region which is between the antigen binding domain and the transmembrane domain. The CARs identified by the invention may comprise an extracellular spacer domain but is it also possible to omit such a spacer. The spacer may include Fc fragments of antibodies or fragments thereof, hinge regions of antibodies or fragments thereof, CH2 or CH3 regions of antibodies, accessory proteins, artificial spacer sequences or combinations thereof. A prominent example of a spacer is the CD8alpha hinge.

The transmembrane domain of the CAR can be derived from any desired natural or synthetic source for such domain. If the source is natural the domain may be derived from any membrane-bound or transmembrane protein.

The cytoplasmic domain or the intracellular signaling domain of the CAR of the invention is responsible for activation of at least one of the normal effector functions of the immune cell in which the CAR is expressed. "Effector function" means a specialized function of a cell, e.g. in a T cell an effector function may be cytolytic activity or helper activity including the secretion of cytokines. The intracellular signaling domain refers to the part of a protein which transduces the effector function signal and directs the cell expressing the CAR of the invention to perform a specialized function. The intracellular signaling domain may include any complete or truncated part of the intracellular signaling domain of a given protein sufficient to transduce the effector function signal.

Prominent examples of intracellular signaling domains for use in the CARs include the cytoplasmic sequences of the T cell receptor (TCR) and co-receptors that act in concert to initiate signal transduction following antigen receptor engagement.

As for CARs with at least two antigen binding domains, the antigen binding domains may have different functions. The process of the invention reduces the time of development for new CAR cells suitable for tumor treatment which can be provided as "AND"-, "NOT"-, "OR"- type CAR cells.

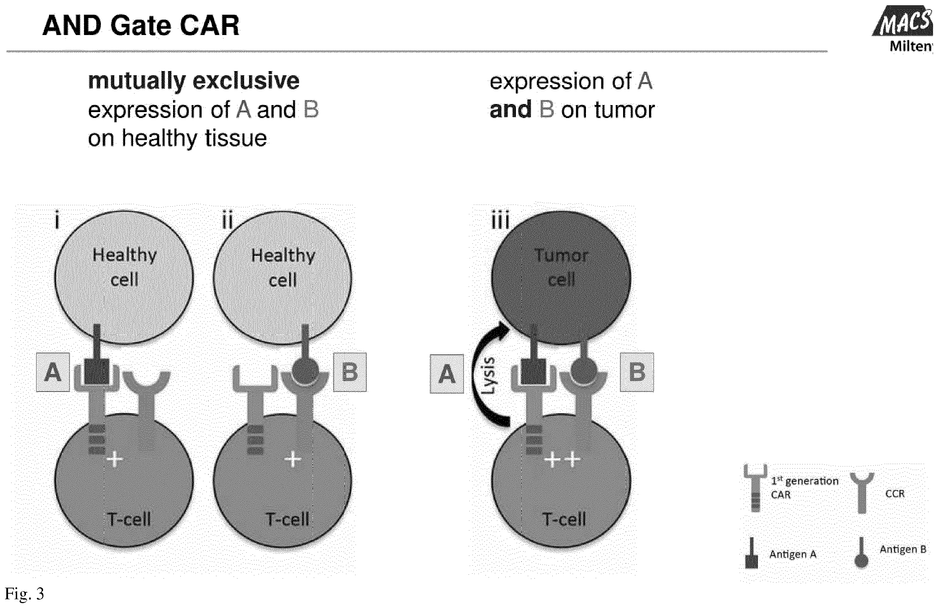

In one embodiment of the invention, the CAR cell will only be activated i.e. the signal of the signal domain will only be triggered when both antigen binding domains bind to their respective antigens. Such CAR cells are hereinafter referred to as "AND-CAR". The antigen binding domains of "AND-CAR" cells may be identified by the method present invention by identifying for example two antigens located on tumor cells of which non or at most one is expressed on healthy cells. Fig. 3 (published in Transl Cancer Res 2016;5(S 1):S61-S65) shows how AND-CARs are deactivated by healthy cells (i) and (ii) but activated by tumor cells (iii).

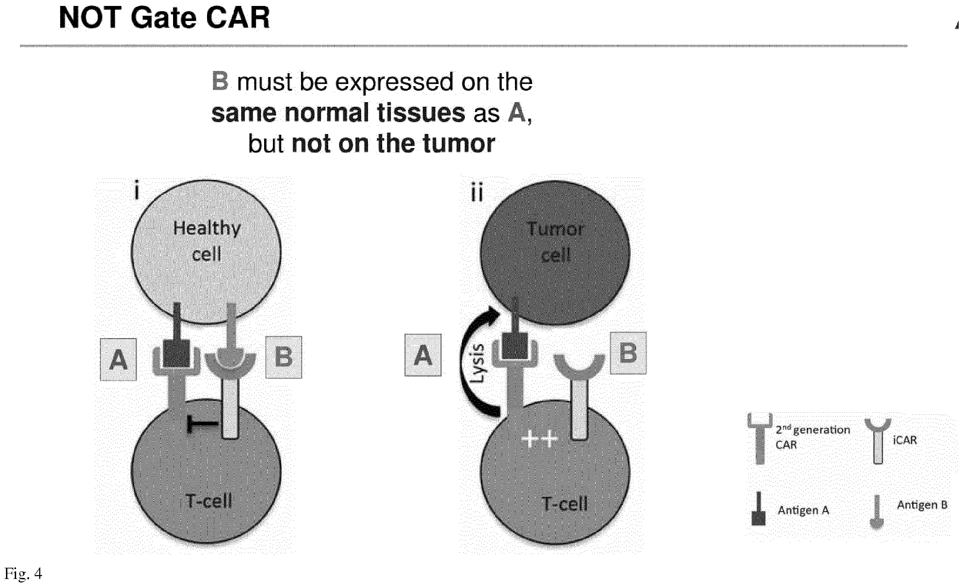

In another embodiment of the invention, the CAR cell will only be activated i.e. the signal of the signal domain will only be triggered when one antigen binding domains binds to its respective antigens and the other does not. Such CAR cells are hereinafter referred to as "NOT-CAR". The antigen binding domains of "NOT-CAR" cells may be identified by the method present invention by identifying for example two antigens located on healthy cells, of which only one is expressed on tumor cells. Fig. 4 (published in Transl Cancer Res 2016;5(S1):S61-S65) shows how NOT-CARs are deactivated by healthy cells (i) but activated by tumor cells (ii.)

In yet another embodiment of the invention, the CAR cell will already be activated i.e. the signal of the signal domain will be triggered when only one of the two antigen binding domains binds to its respective antigens. Such CAR cells are hereinafter referred to as "OR-CAR". The antigen binding domains of "OR-CAR" cells may be identified by the method present invention by identifying for example two antigens located on tumor cells, but not on healthy cells.

A further reduction in processing time can be achieved by using so called "ADAPTER CAR" technology. An "ADAPTER CAR" cell comprises a signaling domain, a transmembrane domain and a linker domain which has no antigen recognizing properties. To this linker domain, one or more antigen recognizing moieties are then conjugated (linker plus molecule = adaptor). Accordingly, in the process according to the invention, the at least two with antigen recognizing moieties as chimeric antigen receptor (CAR) may be provided as ADAPTER-typ.

In a variant of the invention, the cells are provided with the identified at least two antigen recognizing moieties as chimeric antigen receptor (CAR) by providing a cell comprising biotin as antigen recognizing moiety of a chimeric antigen receptor (CAR) and providing conjugates of the identified at least two antigen recognizing moieties with an anti-Biotin moiety and conjugating said conjugates with said cells.

In another variant of the invention, the cell are provided with the identified at least two antigen recognizing moieties as chimeric antigen receptor (CAR) by providing a cell comprising biotin as antigen recognizing moiety of a first chimeric antigen receptor (CAR) and a second epitope as antigen recognizing moiety of a second chimeric antigen receptor (CAR) and providing conjugates of the identified at least two antigen recognizing moieties with an anti-Biotin moiety and a second moiety conjugating said conjugates with said cells.

Suitable linkers are molecules which are either not present in a mammalian (human) body or have a low affinity to naturally occurring antibodies in a mammalian (human) body. Suitable are for example biotin units or modified biotin which are identified by an appropriate adapter like an anti-biotin antibody which is conjugated to the desired conjugated to the antigen recognizing moiety.

"ADAPTER CAR" technology is disclosed in PCT/EP2017/077545 and uses the terms "linker/label epitope" (LLE) for the linker bound to the CAR cell

Preferable, the LLE does not evoke an immune reaction in a subject intended to be treated with cells expressing the CAR. This can be achieved by using an extracellular LLE binding domain of a CAR that is derived from an naturally occurring epitope recognizing molecule such that the LLE is bound with a higher preference than to the endogenous label moiety without linker moiety, i.e. the naturally occurring molecule in the subject (the self antigen).

For example, an extracellular LLE binding domain of a CAR binds with an at least twofold, preferentially at least 5-fold, more preferentially at least 10-fold higher affinity to said LLE than to the said naturally occurring molecule.

In the case of "ADAPTOR" CAR cells, the CAR cell provided with the linker unit and the adaptor are applied in parallel to the patient. Advantage of this embodiment is that the CAR cell with a linker may be prepared independent of the identified antigen recognizing moieties and does not have to be developed from the scratch. Only the adaptor has to be generated and tested. This approach allows also to control the strength and kinetics of the therapy, similar to a conventional chemotherapy.

### Detecting process

In order to investigate a plurality of potential binders or conjugates, it is preferred to quench the fluorescence radiation emitted by the fluorescent moieties by enzymatically degrading of the conjugates. Such conjugates are for example disclosed in EP3037821A1, EP16203689.1 or EP 16203607.3.

In alternative, the fluorescence radiation emitted by the fluorescent moieties is quenched by radiation.

### Use for treatment of cancer

The cancer to be treated may include hematopoietic cancer, myelodysplastic syndrome, pancreatic cancer, head and neck cancer, cutaneous tumors, minimal residual disease (MRD) in acute lymphoblastic leukemia (ALL), acute myeloid leukemia (AML), lung cancer, breast cancer, ovarian cancer, prostate cancer, colon cancer, melanoma or other hematological cancer and solid tumors, or any combination thereof. In another embodiment, the cancer includes a hematological cancer such as leukemia (e.g., chronic lymphocytic leukemia (CLL), acute lymphocytic leukemia (ALL), acute myeloid leukemia (AML), or chronic myelogenous leukemia (CML), lymphoma (e.g., mantle cell lymphoma, non-Hodgkin's lymphoma or Hodgkin's lymphoma) or multiple myeloma, or any combination thereof. Furthermore, the cancer may include an adult carcinoma comprising coral and pharynx cancer (tongue, mouth, pharynx, head and neck), digestive system cancers (esophagus, stomach, small intestine, colon, rectum, anus, liver, intrahepatic bile duct, gallbladder, pancreas), respiratory system cancers (larynx, lung and bronchus), bones and joint cancers, soft tissue cancers, skin cancers (melanoma, basal and squamous cell carcinoma), pediatric tumors (neuroblastoma, rhabdomyosarcoma, osteosarcoma, Ewing's sarcoma), tumors of the central nervous system (brain, astrocytoma, glioblastoma, glioma), and cancers of the breast, the genital system (uterine cervix, uterine corpus, ovary, vulva, vagina, prostate, testis, penis, endometrium), the urinary system (urinary bladder, kidney and renal pelvis, ureter), the eye and orbit, the endocrine system (thyroid), and the brain and other nervous system, or any combination thereof.

The treatment of cancer may encompass any method which involves at least one antigen as disclosed or any combination of antigens as disclosed as target molecule. Such methods may be e.g. treatment with agents which bind to the antigen and affect the viability of the cancerous cell expressing this antigen, preferentially kill the cancerous cell. Examples are oncolytic viruses, BiTEs^{®}, ADCCs and immunotoxins as already disclosed.

For the treatment, immune cells, e.g. T cells of a subject may be isolated. The subject may suffer from said cancer or may be a healthy subject. These cells are genetically modified in vitro or in vivo to express one or more CARs of the invention. These engineered cells may be activated and expanded in vitro or in vivo. In a cellular therapy these engineered cells may be infused to a recipient in need thereof. The infused cells are able to kill (or at least stop growth of) cancerous cells expressing one or more of the disclosed antigens in the recipient. The recipient may be the same subject from which the cells was obtained (autologous cell therapy) or may be from another subject of the same species (allogeneic cell therapy).

For example, cells with the following antigen recognizing moieties as chimeric antigen receptors (CAR) may be used to treat certain cancer types:
a) CLA, TSPAN8 as positive marker which are exclusive for tumor, e.g. pancreatic tumor
b) CLA as positive marker and CD45 as NOT marker to discriminate tumor from healthy cells e.g. pancreatic tumor
c) Epcam and CD90 as positive marker which are exclusive for tumor, e.g. ovarian cancer
d) SSEA4 as positive and CD34 and CD133 as negative marker to discriminate tumor from healthy cells e.g. breast cancer

### Examples

The following examples are intended for a more detailed explanation of the invention but without restricting the invention to these examples.

### Example 1: Identification of combinations of conjugates comprising a fluorescent moiety and an antigen recognizing moiety which in combination allow to discriminate between pancreas carcinoma (PaCa) cells and and non-PaCa cells.

A pancreas carcinoma (PaCa) from a patient was analyzed by subsequent staining with different antibody fluorochrome conjugates, imaging and de-staining.

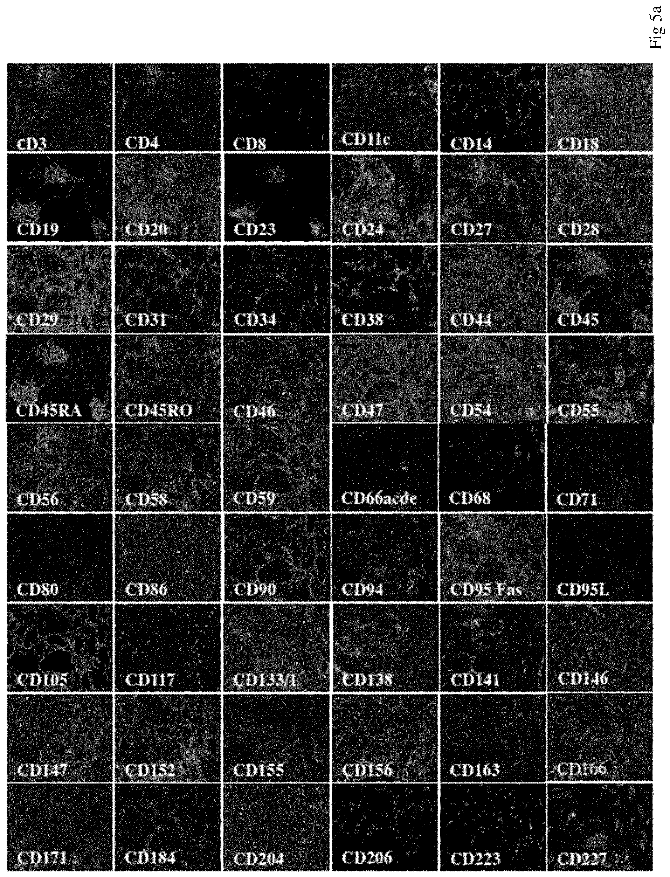

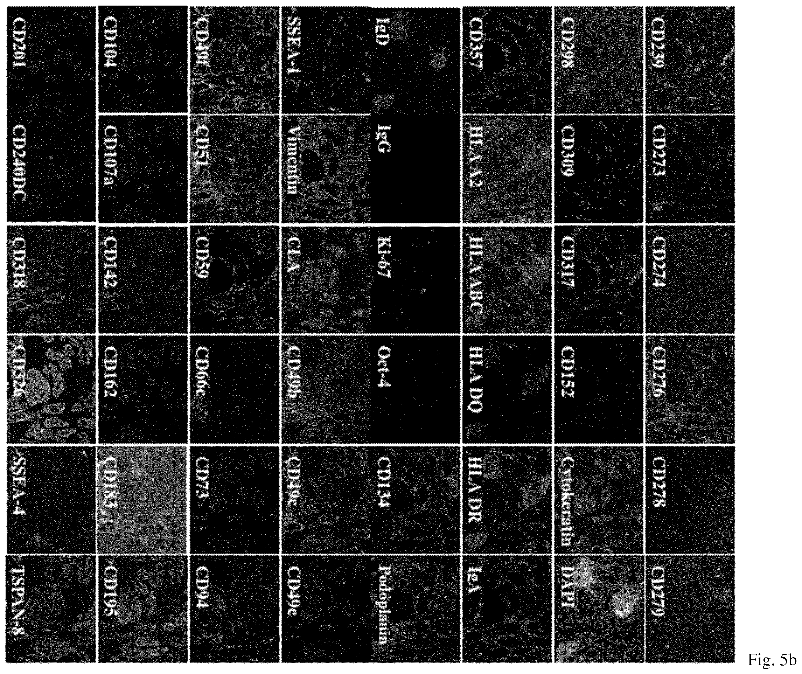

Cryosections of the PaCa sample were fixed and each specimen was stained with 101 fluorescently labeled antibodies and the nuclear labeling dye DAPI (Fig 5). The thus obtained images were analyzed for antigen quantification and pattern recognition at the level of single pixels and segmented cells.

To narrow down the selection of potential targets, antibody conjugates were selected which displayed a similar staining pattern as observed when staining the same specimen with an anti-EpCAM+ (CD326) antibody fluorochrome conjugate. EpCAM is well known in field for its expression on a variety of human epithelial tissues and carcinomas. This applied, for example, to CLA, CD66c, CD318 and TSPAN8 as shown in Fig 5.

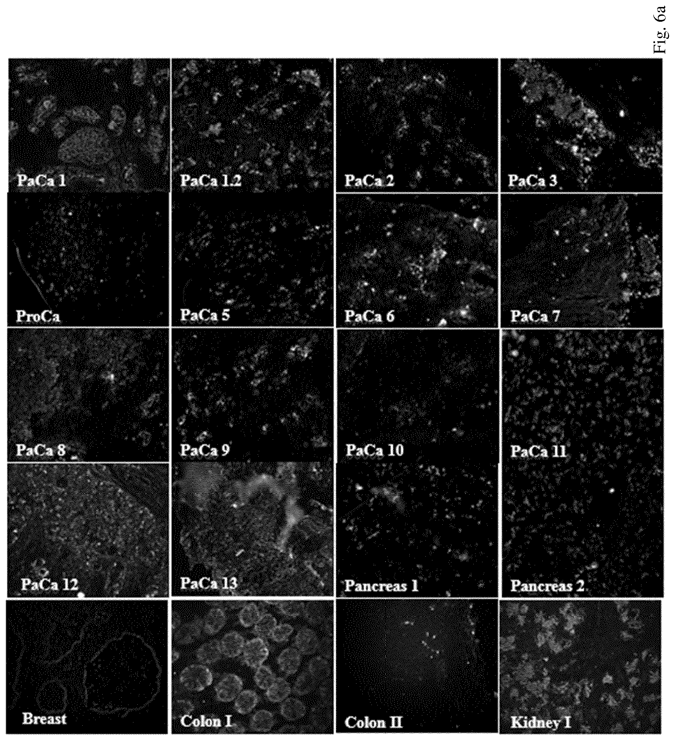

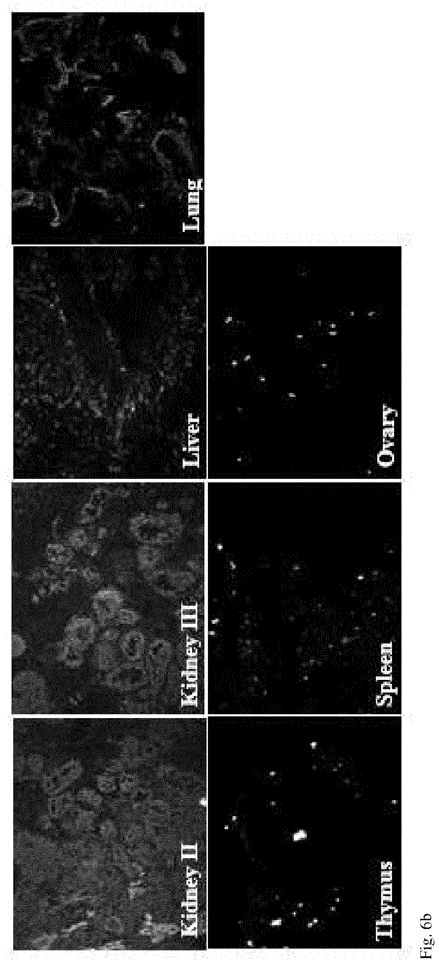

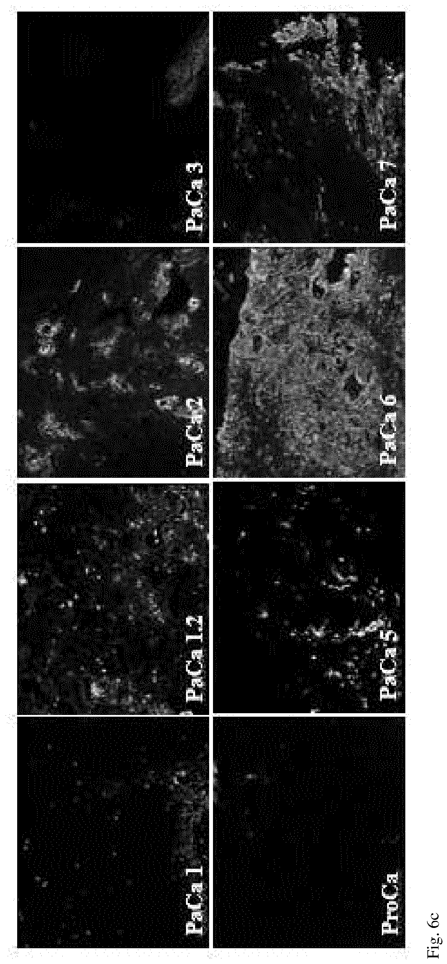

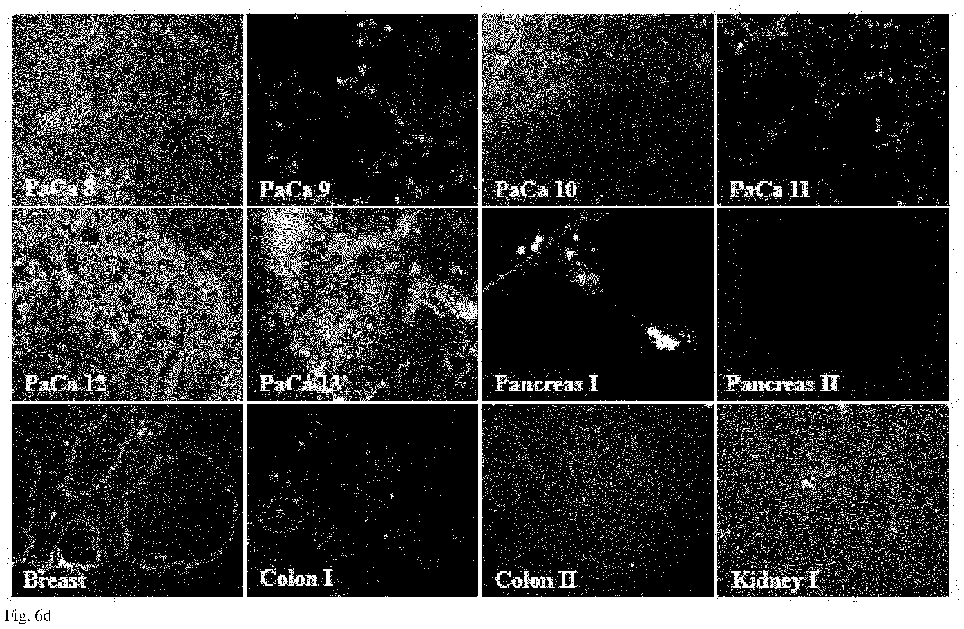

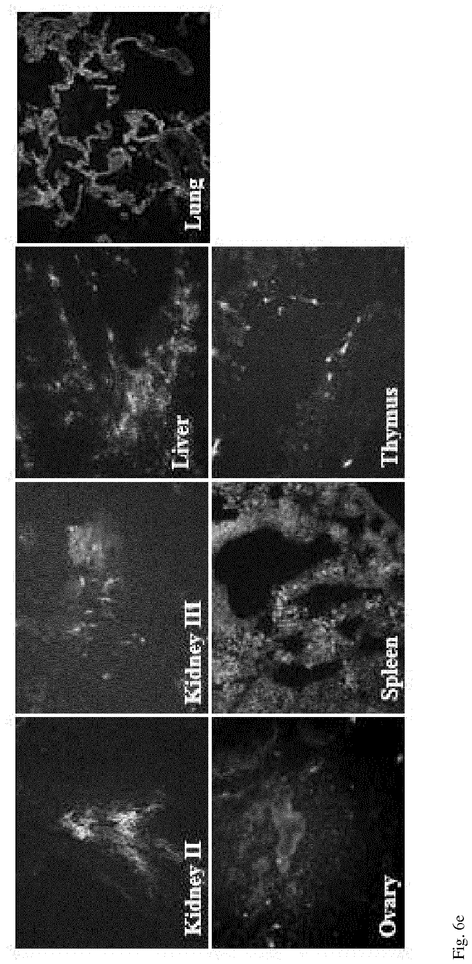

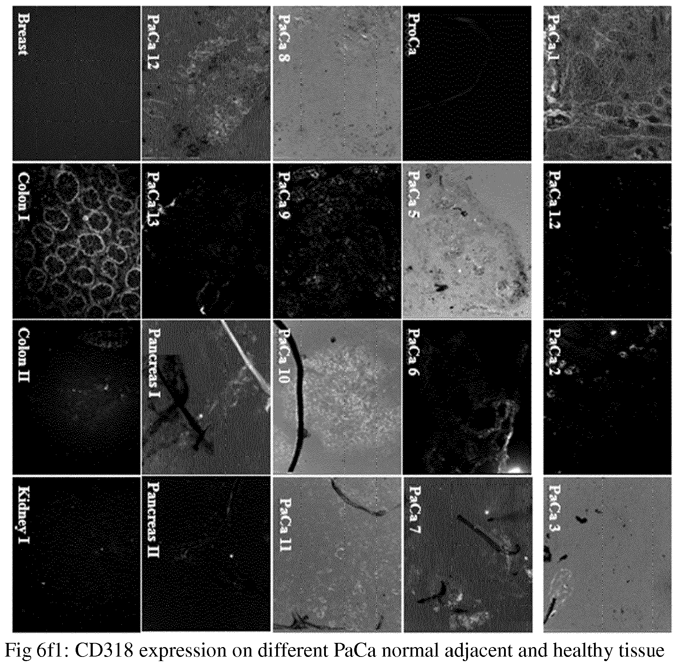

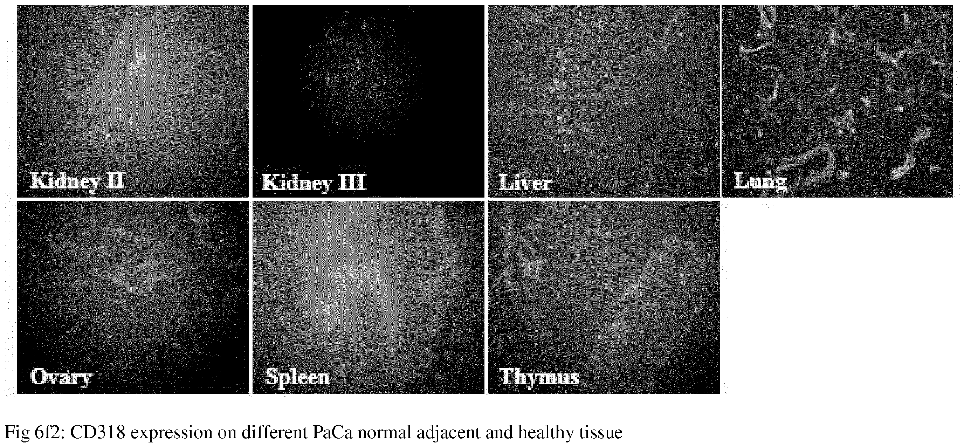

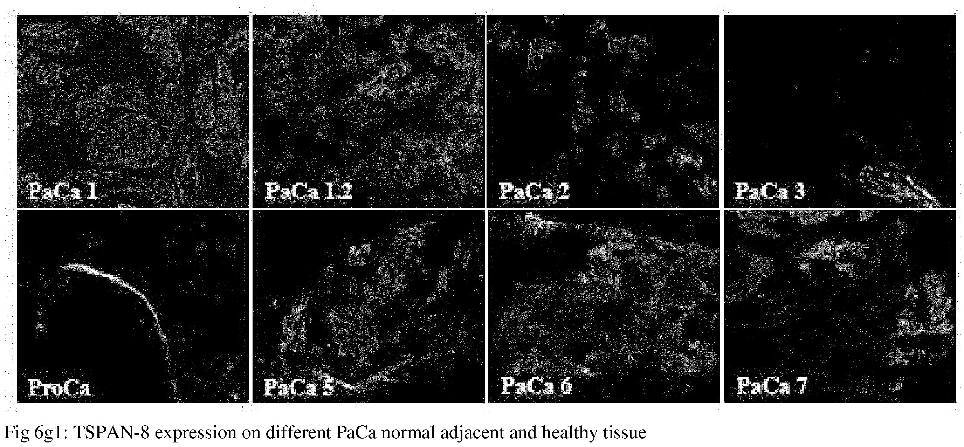

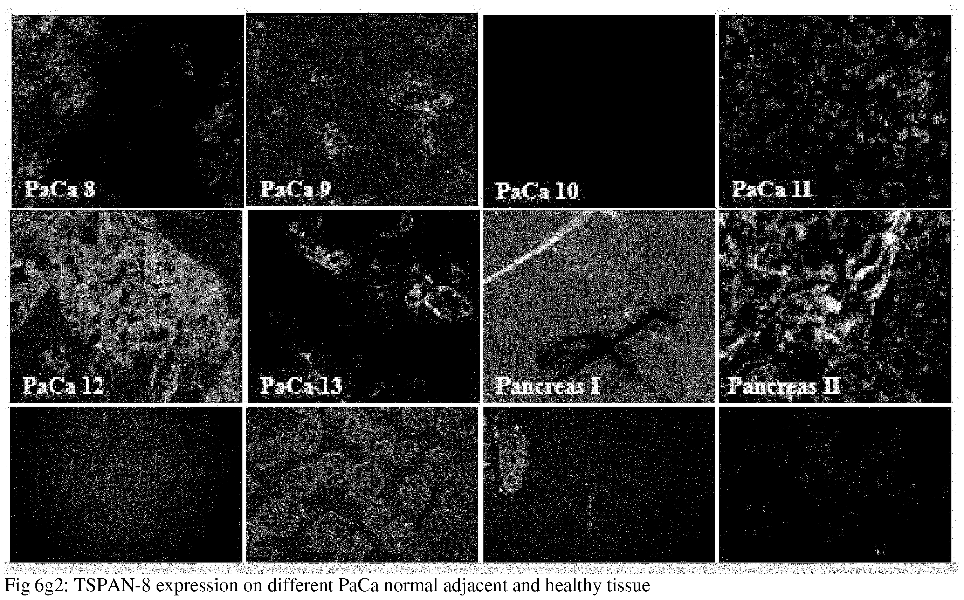

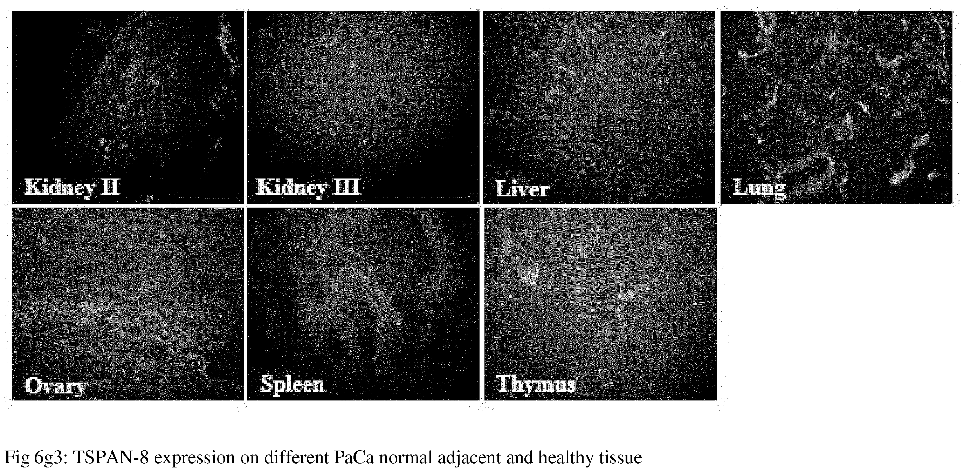

Subsequently, 20 fluorochrome conjugated antibodies of potential targets such as CLA, CD66c, CD318 and TSPAN8 were evaluated for their staining pattern on 16 healthy or normal adjacent (NAT) tissues in the same staining/imaging/de-staining process with the results shown in Fig 6. This was conducted in order to evaluate the off tumor expression of the antigens of interest, thus allowing the prediction of potential on-target off-tumor toxicity of the target candidates. Examples of this analysis are shown in Fig 6 a-c for the markers CLA, CD66c, CD318 and TSPAN8.

The previous experiments show that these markers are expressed on a PaCa sample, it was now investigated if these markers are also expressed on healthy tissue. For example CD318 is also found besides others on colon, lung and liver tissue. Therefore, using CD318 as a single marker to direct CAR T cells would most likely lead to an on-target off-tumor toxicity in the indicated tissues.

Accordingly, the data of the previous experiments was analyzed to find markers which show an expression on the same pancreatic carcinoma specimen but do not show an expression on the same healthy tissue specimen. A number of combinations could be identified as shown in Fig 6 a - c by visual comparison of images or bioinformatic analysis. Visual comparison means that if two images show substantially the same expression pattern of two different conjugates, the respective markers are expressed on substantially the same cells. On the other hand, if two images show different expression patterns for two different conjugates, the respective markers are not expressed on the same cells. For example, CD318 and CLA do not show a combined expression on a healthy tissue specimens.

Finally, the resulting target candidates were tested on a multi patient sample array with 12 different PaCa samples and 1 ProCa sample to evaluate the broadness of applicability of these marker combinations. Here it was shown that the markers are coexpressed on a majority of pancreatic cancers.

Taken together CLA, CD66c, CD318 and TSPAN8 were identified as being candidates for an CAR T cell approach to eradicate a pancreatic cancer with minimal risk of affecting healthy tissue.

### Example 2: Identification of combinations of conjugates comprising a fluorescent moiety and an antigen recognizing moiety which in combination allow to discriminate between ovarian carcinoma (OvCa) cells and and non-OvCa cells.

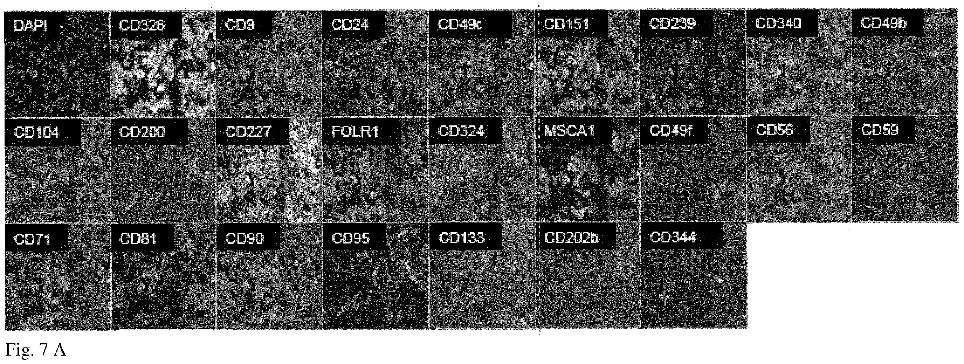

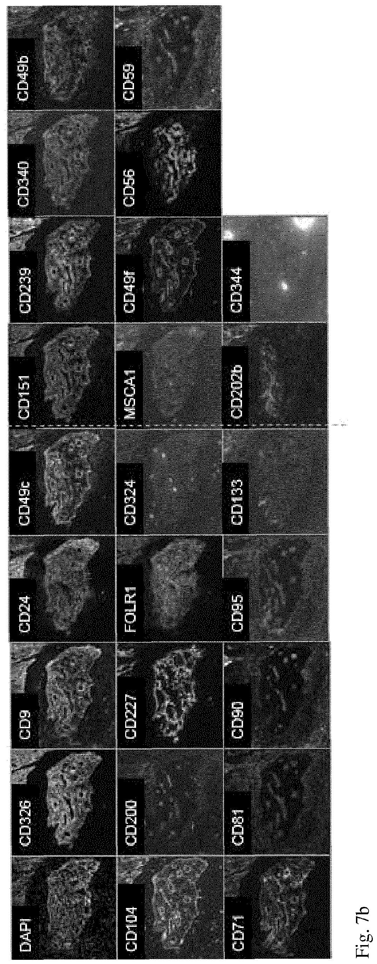

Fresh-frozen high-grade serous ovarian carcinoma resections were sliced and fixed with acetone. A screening was performed using a novel high content imaging platform enabling fully automated cyclic fluorescence imaging of individual biological samples. Sequential staining of two OvCa specimen with 20 antigen recognizing moiety conjugated to a fluorescent mojety revealed the expression of a number of antigens. Besides EpCAM (CD326), which is a well-known marker for human epithelial tissues and carcinomas, these were for example FOLR1, CD227, and CD340 (Fig 7).

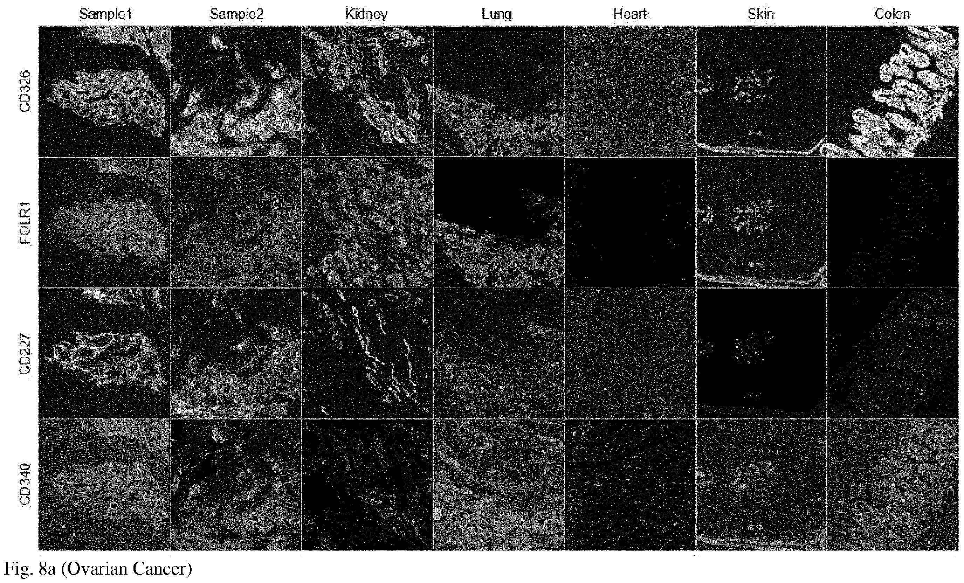

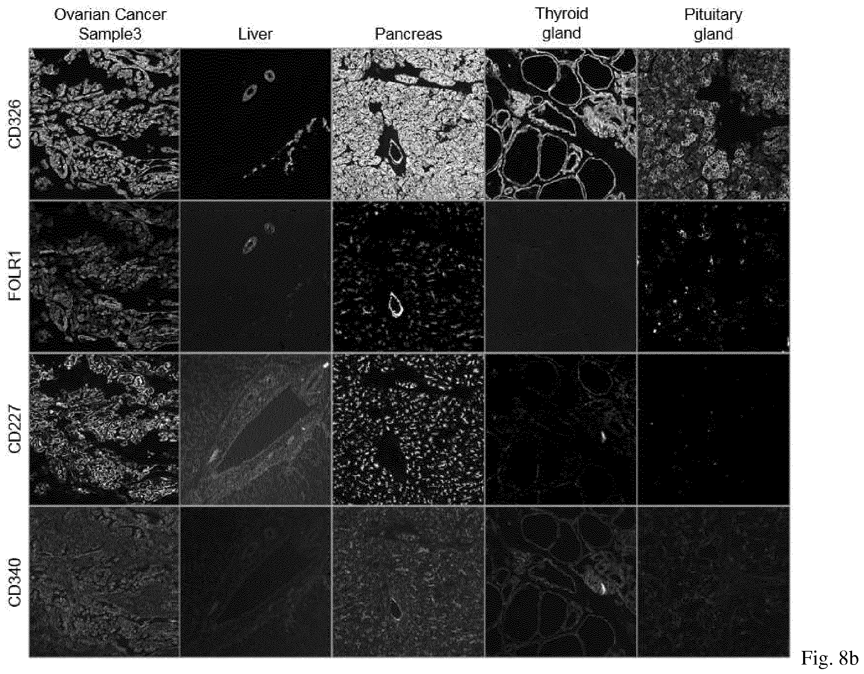

The target candidates CD326 FOLR1, CD227, and CD340 were validated using the staining/imaging/de-staining process by comparing their expression levels on two independent high-grade serous ovarian carcinoma resection samples as well as healthy human tissue samples (kidney, lung, heart, skin, and colon) (Fig 8a) and on a third independent high-grade serous ovarian carcinoma resection sample as well as healthy human tissue samples (liver, pancreas, thyroid gland, and pituitary gland) (Fig 8b).

Data analysis and further co-stainings revealed that while the four indicated markers are all expressed on OvCa samples they show also expression on healthy tissue. However, the expression on healthy tissue was found to be at least partially discriminative opening the possibility to combine two or more markers to get a selective labelling of OvCa cells. For example, CD227 and FOLR1 showed both an expression on a majority of OvCa cells. While CD227 showed an additional pronounced expression on liver, lung and pancreas, FOLR1 showed a notable expression on kidney, lung and skin. When combining both markers and interrogating a cellular co-expression it was found that none of the above mentioned healthy tissue showed a cellular co-expression in a substantial proportion of cells. Therefore, the combination of CD227 and FOLR1 presents an option to eradicate OvCa cells with CAR T which are only activated when both antigens CD227 and FOLR1 are bound (AND CAR T cells) with no or strongly reduced on-target off-tumor toxicity. On the other when performing similar analysis as outlined above it was found that for example the combination of FOLR1 and Podoplanin opens the possibility for a NOT CAR T cell treatment of OvCa. While FOLR1 is expressed on OvCa cells but also on kidney, lung and skin, Podoplanin does not show a cellular co-expression with FOLR1 on OvCa cells but is strongly expressed on kidney, lung and skin.

Therefore, using CAR T cells which are activated by the detection of FOLR1 but deactivated or inhibited by the detection of Podoplanin should result in an efficient killing of OvCa cells leaving healthy cells unaffected either because FOLR1 is not expressed or Podoplanin is expressed.

## Claims

1. Process for providing a cell comprising a chimeric antigen receptor (CAR) specific for one or more target antigens exposed on tumor cells **characterized by** providing a cell sample comprising tumor and non-tumor cells and repeating the steps of
- contacting the cell tissue with a conjugate comprising a fluorescent moiety and an antigen recognizing moiety
- removing unbound conjugate from the cell tissue and detecting cells bound to the conjugate by the fluorescence radiation emitted by the fluorescent moieties of the first conjugates
- erasing the fluorescence emitted by the fluorescent moieties of the conjugates with conjugates comprising a fluorescent moiety and antigen recognizing moieties recognizing different antigens
until identifying at least two conjugates provided with antigen recognizing moieties recognizing different antigens, allowing in combination to discriminate between tumor cells and non-tumor cells and
providing cells with the identified at least two antigen recognizing moieties as chimeric antigen receptor (CAR).

2. Process according to claim 1, **characterized in that** the cell comprising a chimeric antigen receptor (CAR) is selected from the group consisting of T-cells, NK-cells, or cells engineered to destroy other cells when triggered by binding of the other cell.

3. Process according to claim 1 or 2, **characterized in that** the at least two antigen recognizing moieties are provided as AND-, OR- or NOT-type.

4. Process according to any of the claims 1 to 3, **characterized in that** the at least two antigen recognizing moieties are provided as ADAPTER-type.

5. Process according to claim 4 **characterized in that** the cell are provided with the identified at least two antigen recognizing moieties as chimeric antigen receptor (CAR) by providing a cell comprising biotin as antigen recognizing moiety of a chimeric antigen receptor (CAR) and providing conjugates of the identified at least two antigen recognizing moieties with an anti-Biotin moiety and conjugating said conjugates with said cells.

6. Process according to claim 4 **characterized in that** the cell are provided with the identified at least two antigen recognizing moieties as chimeric antigen receptor (CAR) by providing a cell comprising biotin as antigen recognizing moiety of a first chimeric antigen receptor (CAR) and a second epitope as antigen recognizing moiety of a second chimeric antigen receptor (CAR) and providing conjugates of the identified at least two antigen recognizing moieties with an anti-Biotin moiety and a second moiety conjugating said conjugates with said cells.

7. Process according to any of the claims 1 to 6, **characterized in that** the at least two conjugates provided with antigen recognizing moieties binding at least at least 20% of the tumor cells and less than 5% of the non-tumor cells.

8. Process according to any of the claims 1 to 7, **characterized in that** the at least two conjugates provided with antigen recognizing moieties binding at least 10 fold more tumor cells than non-tumor cells of the cell sample.

9. Process according to any of the claims 1 to 8, **characterized in that** the fluorescence radiation emitted by the fluorescent moieties is erased by enzymatically degrading of the conjugates.

10. Process according to any of the claims 1 to 8, **characterized in that** the fluorescence radiation emitted by the fluorescent moieties is erased by radiation.

## Patentansprüche

1. Verfahren zur Bereitstellung einer Zelle, die einen chimären Antigenrezeptor (CAR) umfasst, der für ein oder mehrere Zielantigene spezifisch ist, die auf Tumorzellen exponiert sind, das durch das Bereitstellen einer Zellprobe, die Tumor- und Nicht-Tumor-Zellen umfasst, und das Wiederholen der folgenden Schritte gekennzeichnet ist:
- Inkontaktbringen des Zellgewebes mit einem Konjugat, das eine fluoreszierende Komponente und eine Antigen erkennende Komponente umfasst,
- Entfernen von ungebundenem Konjugat aus dem Zellgewebe und Nachweisen von Zellen, die an das Konjugat gebunden sind, durch die Fluoreszenzstrahlung, die von den fluoreszierenden Komponenten der ersten Konjugate emittiert werden,
- Entfernen der von den fluoreszierenden Komponenten der Konjugate emittierten Fluoreszenz mit Konjugaten, die eine fluoreszierende Komponente und Antigen erkennende Komponenten umfassen, die unterschiedliche Antigene erkennen,
bis zum Identifizieren von mindestens zwei Konjugaten, die mit Antigen erkennenden Komponenten bereitgestellt werden, die unterschiedliche Antigene erkennen, die in Kombination eine Unterscheidung zwischen Tumorzellen und Nicht-Tumorzellen ermöglichen, und Bereitstellen von Zellen mit den identifizierten mindestens zwei antigenerkennenden Komponenten als chimärem Antigenrezeptor (CAR).

2. Verfahren nach Anspruch 1, **dadurch gekennzeichnet, dass** die Zelle, die einen chimären Antigenrezeptor (CAR) umfasst, aus der Gruppe ausgewählt ist, die aus T-Zellen, NK-Zellen oder Zellen besteht, die so konstruiert sind, dass sie andere Zellen zerstören, wenn sie durch Bindung der anderen Zelle dazu stimuliert werden.

3. Verfahren nach Anspruch 1 oder 2, **dadurch gekennzeichnet, dass** die mindestens zwei Antigen erkennenden Komponenten als UND-, ODER- oder NICHT-Typ bereitgestellt werden.

4. Verfahren nach einem der Ansprüche 1 bis 3, **dadurch gekennzeichnet, dass** die mindestens zwei Antigen erkennenden Komponenten als ADAPTER-Typ vorgesehen sind.

5. Verfahren nach Anspruch 4, **dadurch gekennzeichnet, dass** die Zelle mit den identifizierten mindestens zwei Antigen erkennenden Komponenten als chimärer Antigenrezeptor (CAR) bereitgestellt wird, indem eine Zelle bereitgestellt wird, die Biotin als Antigen erkennende Komponente eines chimären Antigenrezeptors (CAR) umfasst, und Konjugate der identifizierten mindestens zwei Antigen erkennenden Komponenten mit einer Anti-Biotin-Komponente bereitgestellt werden und die Konjugate mit den Zellen konjugiert werden.

6. Verfahren nach Anspruch 4, **dadurch gekennzeichnet, dass** die Zelle mit den identifizierten mindestens zwei Antigen erkennenden Komponenten als chimärer Antigenrezeptor (CAR) bereitgestellt wird, indem eine Zelle bereitgestellt wird, die Biotin als Antigen erkennende Komponente eines ersten chimären Antigenrezeptors (CAR) und ein zweites Epitop als Antigen erkennende Komponente eines zweiten chimären Antigenrezeptors (CAR) umfasst, und Konjugate der identifizierten mindestens zwei Antigen erkennenden Komponenten mit einer Anti-Biotin-Komponente und einer zweiten Komponente bereitgestellt werden, die die Konjugate mit den Zellen konjugieren.

7. Verfahren nach einem der Ansprüche 1 bis 6, **dadurch gekennzeichnet, dass** die mindestens zwei Konjugate, die mit Antigen erkennenden Komponenten bereitgestellt werden, mindestens 20 % der Tumorzellen und weniger als 5 % der Nicht-Tumorzellen binden.

8. Verfahren nach einem der Ansprüche 1 bis 7, **dadurch gekennzeichnet, dass** die mindestens zwei Konjugate, die mit Antigen erkennenden Komponenten bereitgestellt werden, mindestens 10-mal mehr Tumorzellen als Nicht-Tumorzellen der Zellprobe binden.

9. Verfahren nach einem der Ansprüche 1 bis 8, **dadurch gekennzeichnet, dass** die Fluoreszenzstrahlung, die von den fluoreszierenden Komponenten emittiert wird, durch enzymatischen Abbau der Konjugate entfernt wird.

10. Verfahren nach einem der Ansprüche 1 bis 8, **dadurch gekennzeichnet, dass** die Fluoreszenzstrahlung, die von den fluoreszierenden Komponenten emittiert wird, durch Strahlung entfernt wird.

## Revendications

1. Procédé de fourniture d'une cellule comprenant un récepteur d'antigène chimérique (CAR) spécifique pour un ou plusieurs antigènes cibles exposés sur des cellules tumorales **caractérisé** comme fournissant un échantillon de cellules comprenant des cellules tumorales et non tumorales et répétant les étapes consistant à
- mettre en contact le tissu cellulaire avec un conjugué comprenant une fraction fluorescente et une fraction de reconnaissance d'antigène
- enlever le conjugué non lié du tissu cellulaire et détecter les cellules liées au conjugué par le rayonnement de fluorescence émis par les fractions fluorescentes des premiers conjugués
- effacer la fluorescence émise par les fractions fluorescentes des conjugués avec des conjugués comprenant une fraction fluorescente et des fractions de reconnaissance d'antigène reconnaissant différents antigènes
jusqu'à identifier au moins deux conjugués fournis avec des fractions de reconnaissance d'antigène reconnaissant différents antigènes, permettre la combinaison pour discriminer entre les cellules tumorales et non tumorales et
fournir des cellules avec les au moins deux fractions de reconnaissance d'antigène identifiées comme récepteurs d'antigène chimérique (CAR).

2. Procédé selon la revendication 1, **caractérisé en ce que** la cellule qui comprend un récepteur d'antigène chimérique (CAR) est choisie dans le groupe constitué par les lymphocytes T, les cellules NK, ou des cellules modifiées pour détruire d'autres cellules quand elles sont déclenchées par la liaison de l'autre cellule.

3. Procédé selon la revendication 1 ou 2, **caractérisé en ce que** les au moins deux fractions de reconnaissance d'antigène sont fournies comme le type ET-, OU- ou NON-.

4. Procédé selon l'une quelconque des revendications 1 à 3, **caractérisé en ce que** les au moins deux fractions de reconnaissance d'antigène sont fournies comme le type ADAPTER.

5. Procédé selon la revendications 4 **caractérisé en ce que** les cellules sont fournies avec les au moins deux fractions de reconnaissance d'antigène identifiées comme récepteurs d'antigène chimérique (CAR) en fournissant une cellule comprenant de la biotine comme fraction de reconnaissance d'antigène d'un récepteur d'antigène chimérique (CAR) et en fournissant des conjugués des au moins deux fractions de reconnaissance d'antigène identifiées avec une fraction anti-biotine et en conjuguant lesdits conjugués avec lesdites cellules.

6. Procédé selon la revendication 4 **caractérisé en ce que** les cellules sont fournies avec les au moins deux fractions de reconnaissance d'antigène identifiées comme récepteurs d'antigène chimérique (CAR) en fournissant une cellule comprenant de la biotine comme fraction de reconnaissance d'antigène d'un premier récepteur d'antigène chimérique (CAR) et un second épitope comme fraction de reconnaissance d'antigène d'un second récepteur d'antigène chimérique (CAR) et en fournissant des conjugués des au moins deux fractions de reconnaissance d'antigène identifiées avec une fraction anti-biotine et une seconde fraction conjuguant lesdits conjugués avec lesdites cellules.

7. Procédé selon l'une quelconque des revendications 1 à 6, **caractérisé en ce que** les au moins deux conjugués fournis avec les fractions de reconnaissance d'antigène se liant à au moins 20 % des cellules tumorales et à moins de 5 % des cellules non tumorales.

8. Procédé selon l'une quelconque des revendications 1 à 7, **caractérisé en ce que** les au moins deux conjugués fournis avec les fractions de reconnaissance d'antigène se liant avec au moins 10 fois plus de cellules tumorales que de cellules non tumorales de l'échantillon de cellules.

9. Procédé selon l'une quelconque des revendications 1 à 8, **caractérisé en ce que** le rayonnement de fluorescence émis par les fractions fluorescentes est effacé par dégradation enzymatique des conjugués.

10. Procédé selon l'une quelconque des revendications 1 à 8, **caractérisé en ce que** le rayonnement de fluorescence émis par les fractions fluorescentes est effacé par un rayonnement.
